Europäisches Patentamt

(19) European Patent Office

Office européen des brevets

(11) Numéro de publication: **0 043 317**
**B1**

(12) **FASCICULE DE BREVET EUROPEEN**

(45) Date de publication du fascicule du brevet:
03.10.84

(21) Numéro de dépôt: 81401009.6

(22) Date de dépôt: 23.06.81

(51) Int. Cl.³: **C 08 G 18/83,** C 08 G 18/08,
A 61 L 15/01, A 61 L 15/00,
A 61 K 7/48, A 61 K 31/785

(54) Procédé de préparation d'un polymère de structure tridimensionnelle du type polyuréthane réticulé, produit obtenu selon ce procédé et son application en tant qu'agent gonflant, notamment en thérapeutique.

(30) Priorité: 30.06.80 FR 8014515

(43) Date de publication de la demande:
06.01.82 Bulletin 82/1

(45) Mention de la délivrance du brevet:
03.10.84 Bulletin 84/40

(84) Etats contractants désignés:
AT BE CH DE FR GB IT LI LU NL SE

(56) Documents cités:
FR - A - 2 130 202
US - A - 4 127 516

(73) Titulaire: **LABORATOIRES D'HYGIENE ET DE DIETETIQUE L.H.D. Société Anonyme dite:, 3 rue de Citeaux, F-75012 Paris (FR)**

(72) Inventeur: **Grouiller, Hervé, 1 rue d'Aval, à Chevrey, F-21700 Arcenant (FR)**

(74) Mandataire: **Richebourg, Michel François et al, Cabinet Beau de Loménie 55, rue d'Amsterdam, F-75008 Paris (FR)**

## Description

La présente invention a trait à un procédé de préparation d'un polymère de structure tridimensionnelle du type polyuréthane réticulé. Elle concerne également le polymère obtenu selon ce procédé, en tant que produit industriel nouveau, et son application en tant qu'agent gonflant utile notamment en thérapeutique, en particulier en tant qu'agent anorexigène, hypolipidémiant, hypocholestérolémiant et séquestrant des acides biliaires.

On sait que l'on a déjà proposé dans le passé des substances macromoléculaires de structure tridimensionnelle ayant un pouvoir gonflant et utiles notamment en tant qu'agents échangeurs d'ions, agents de filtration moléculaires, et agents réduisant le taux de cholestérol et des lipides et/ou agents séquestrant les acides biliaires, voir à cet effet FR-A-1 363 978, FR-A-1 473 582, FR-A-2 140 203 et FR-A-2 140 204. En particulier, FR-A-2 140 203 et FR-A-2 140 204 ont trait à des produits insolubles dans l'eau et les solvants organiques mais qui gonflent dans l'eau et lesdits solvants organiques suivant une forme gélifiée, ces produits gonflants étant obtenus par cocondensation d'une polyamine, l'hexaméthylènediamine ou la xylylènediamine, avec un composé bifonctionnel, notamment du type épichlorhydrine ou 1,3-glycéroldiglycide-éther.

Selon l'invention, on préconise une solution technique différente pour résoudre le problème de l'obtention d'une substance macromoléculaire de structure tridimensionnelle et capable de gonfler en présence de l'eau et des autres solvants. En outre, cette solution technique présente, par rapport à l'art antérieur et en particulier par rapport aux deux demandes françaises précitées, notamment l'avantage d'un meilleur pouvoir gonflant.

Le procédé de préparation selon l'invention d'un polymère de structure tridimensionnelle, du type polyuréthane réticulé, et ayant un bon pouvoir gonflant, est caractérisé en ce que

1. on fait réagir

a) un polyoléther, choisi parmi l'ensemble constitué par les polyols oxyalkylènés (I) et leurs mélanges, obtenu par condensation d'un polyol (II) renfermant au moins 2 groupes OH avec un oxyde d'alkylène (III) à raison de 1 à 20 moles de (III) par groupe OH libre de (II), avec

b) un polyisocyanate (IV) renfermant au moins deux groupes NCO libres, la réaction étant effectuée avec un excès de groupes OH de (I) par rapport aux groupes isocyanates NCO de (IV) pour obtenir un polyuréthane-polyol (V) ayant des groupes OH libres; et

2. on fait réagir le polyuréthane-polyol (V) ainsi obtenu avec l'épichlorhydrine (VI) et une polyamine (VII) choisie parmi les polyamines possédant au moins deux groupes amino $NH_2$ et leurs mélanges.

Parmi les polyoléthers qui conviennent, on peut mentionner ceux qui sont obtenus par réaction (i) d'un polyol tel que le sorbitol, le dulcitol, le pentaérythritol, les alkylèneglycols, notamment l'éthylèneglycol et le propylèneglycol, et d'une manière générale les polyols renfermant de 3 à 6 groupes OH tels que les triols, tétraols, pentols, hexols, ainsi que leurs mélanges, avec (ii) un oxyde d'alkylène (en abrégé OA), notamment l'oxyde d'éthylène et l'oxyde de propylène, à raison de 1 à 20 groupes OA par OH libre de (II).

Parmi les polyisocyanates (IV) qui conviennent selon l'invention, on peut notamment citer les substances renfermant au moins 2 groupes NCO libres, leurs mélanges et leurs prépolymères avec les polyols, lesdits prépolymères renfermant au moins 2 groupes NCO libres par molécule. Parmi les polyisocyanates utilisables, les plus intéressants sont les 2,4-toluène-diisocyanate, 2,6-toluène-diisocyanate, diphénylméthyl-diisocyanate, les polyisocyanates préférés étant le 2,4-toluènediisocyanate et le toluènediisocyanate commercial renfermant 80% en poids d'isomère 2,4 et 20% en poids d'isomère 2,6.

La réaction du stade 1) est effectuée à une température comprise entre 20 et 150°C, de préférence à une température comprise entre 100 et 140°C, pendant au moins 1 heure et de préférence pendant 2 à 3 heures, sous atmosphère d'azote. Les quantités de (I) et de (IV) à utiliser de préférence au stade 1) sont telles que le rapport [NCO]−[OH], où [NCO] représente le nombre total de groupes NCO libres provenant de (IV) et [OH] le nombre total de groupes OH libres provenant de (I), soit compris entre (1:3) et (3:4) et encore plus avantageusement entre (1:2) et (2:3).

Dans ce qui suit les composés (V) obtenus à partir d'un rapport (1:3) ou (3:4) tel que défini ci-dessus, seront appelés polyuréthane-polyol (1:3) et respectivement polyuréthane-polyol (3:4).

A la fin du stade 1) et avant de mettre en œuvre le stade 2), on procède à un gonflement du polyuréthane-polyol (V) au moyen d'un solvant notamment choisi parmi l'eau et les solvants organiques classiques tels que l'acétone, le tétrahydrofuranne, le diméthylformamide, les solvants halogénés notamment le dichloroéthane, le chloroforme, le tétrachlorure de carbone et leurs mélanges. On utilisera de préférence le dichloroéthane, le diméthylformamide ou l'eau, et plus avantageusement parmi ceux-ci, le diméthylformamide.

Le gonflement est effectué de telle façon que le polyuréthane-polyol (V) ait un volume 2 à 20 fois, de préférence 5 à 10 fois, son volume initial. Le gonflement est réalisé sous agitation. Après que le gonflement ait atteint le volume souhaité on broie le polyuréthane-polyol ainsi gonflé en particules les plus fines possibles puis sèche et élimine le solvant de gonflement.

Sans sortir du cadre de l'invention, l'épichlorhydrine, qui est le moyen préféré du stade 2) peut être remplacée par un moyen équivalent, tel que par exemple l'épibromhydrine, les halohydrine et d'une manière générale les composés bifonctionnels décrits dans les brevets précités.

Parmi les polyamines (VII) utilisables au stade 2) on peut notamment mentionner les diamines aliphatiques, les diamines aromatiques et les diamines aralkyliques, en particulier l'hexaméthylènediamine, la phénylènediamine et la xylylènediamine, la diamine préférée étant l'hexaméthylènediamine. Les groupes amino $-NH_2$ du moyen (VII) peuvent, le cas échéant, être quaternisés en groupes $-NH_3^+$.

La réaction du stade 2) sera avantageusement mise en œuvre en utilisant un rapport nombre de groupes OH libres de (V)/nombre de groupes époxydes de l'épichlorhydrine compris entre (1:1) et (1:50) ainsi qu'un rapport nombre de groupes OH libres de (V)/nombre de groupes amino $NH_2$ de (VII) compris entre (1:1) et (1:50).

Au stade 2) on fait réagir soit l'épichlorhydrine avant la polyamine, soit la polyamine avant l'épichlorhydrine, soit encore ces deux moyens en même temps. Du point de vue pratique, on préconise de mettre en œuvre l'épichlorhydrine avant la polyamine.

La réaction du stade 2) est (i) effectuée à une température comprise entre 20 et 100°C (de façon avantageuse entre 40 et 80°C et de préférence à 60–70°C) pendant 2 minutes à 2 h, sous agitation, jusqu'à ce que le milieu réactionnel se gélifie, puis (ii) poursuivie, l'agitation n'étant plus nécessaire, à une température de 60–70°C jusqu'à ce que les composés (VI) et (VII) aient disparu du milieu réactionnel.

Le stade 2) est réalisé en présence d'un solvant choisi parmi l'eau, les solvants organiques classiques tels que l'acétone, le diméthylformamide, le tétrahydrofuranne, les solvants halogénés notamment le dichloroéthane, le chloroforme, le tétrachlorure de carbone, et leurs mélanges. Les solvants préférés sont le dichloréthane et l'eau.

Le produit ainsi obtenu est soumis à un essorage sous vide ou à une aspiration puis séché à 40–80°C (de préférence à 50°C) pendant 24 à 72 h (de préférence 48 h). Le séchage peut être effectué à la pression atmosphérique ou sous vide.

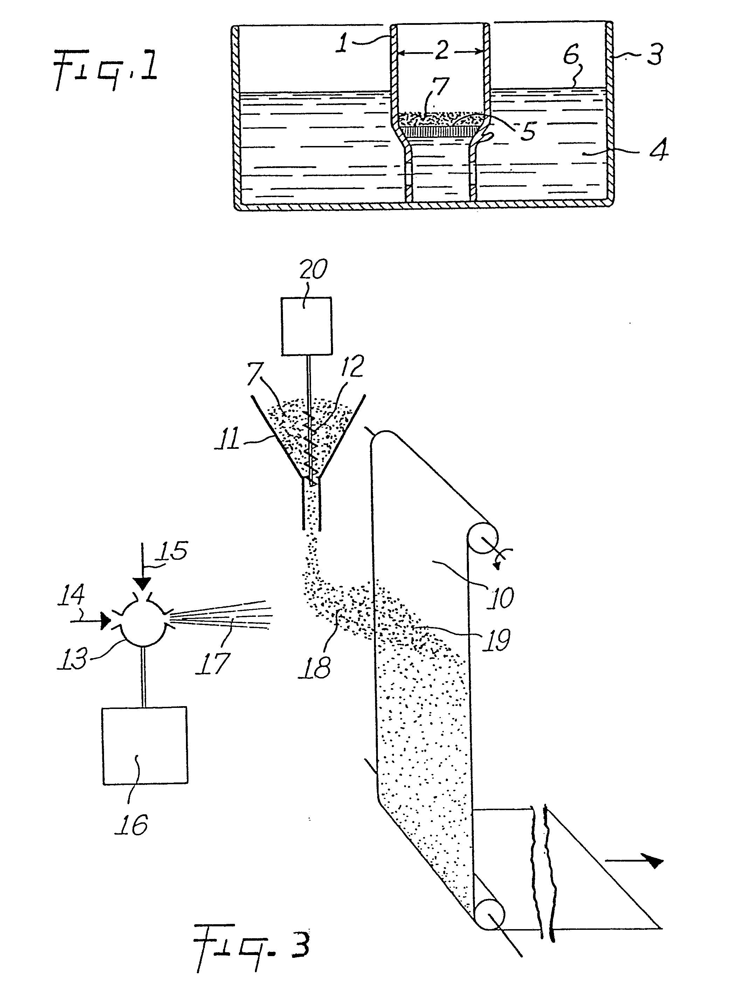

Les essais d'absorption d'eau ont été réalisés au moyen d'un dispositif expérimental permettant de mesurer rapidement l'absorption en fonction du temps, et représenté schématiquement par la fig. 1 ci-après. Ce dispositif comprend un creuset filtre 1 (du type creuset pour filtration en verre fritté No. 3) qui a un diamètre intérieur 2 de 20 mm et qui est placé dans un bac 3 rempli d'eau distillée 4, la partie supérieure du verre fritté 5 étant située à 1 cm au-dessous du niveau 6 de l'eau. Dans ces conditions le creuset filtre est soumis par le bas à une pression d'eau de 1 g/cm² suffisant à provoquer un suintement à travers le verre fritté. 0,5 g de polymère en poudre à étudier est mis au contact de toute la paroi humide 5. La quantité d'eau absorbée est mesurée par pesée de l'ensemble (filtre, polymère, eau absorbée) toutes les 10 secondes.

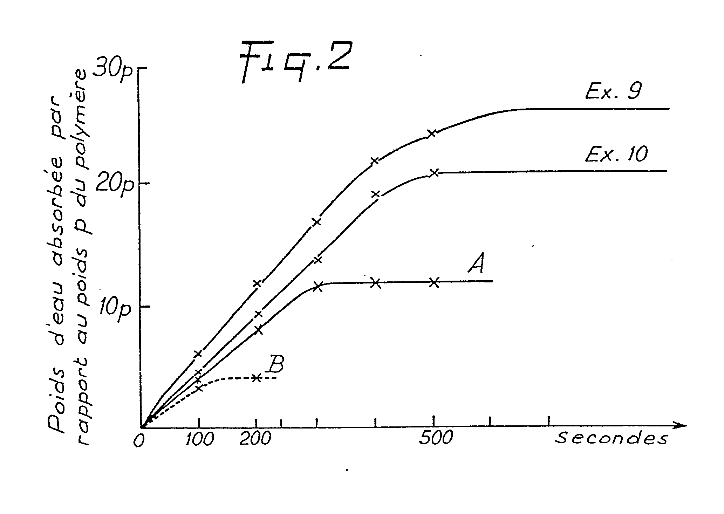

La fig. 2 illustre les résultats obtenus selon cette technique pour les produits des exemples 9 et 10, le produit de l'exemple comparatif A, et un produit de référence B commercialisé en tant qu'agent gonflant sous le nom de Debrisan. Dans la fig. 2 le poids d'eau absorbée par rapport au poids p du polymère est exprimé en fonction du temps donné en secondes.

Dans ce qui suit, on utilise par commodité l'expression «pouvoir hydrophile» pour désigner la valeur maximale de la quantité d'eau absorbée en poids par rapport au poids du polymère.

La détermination du pouvoir plasmophile est réalisée selon la technique exposée ci-dessus pour le pouvoir hydrophile.

Le polymère obtenu selon le procédé de l'invention a un pouvoir hydrophile de 1500 à 4000% en poids (15 à 40 fois son poids d'eau) et a un pouvoir plasmophile de 1200 à 2500% en poids (12 à 25 fois son poids de plasma), alors que les produits des exemples comparatifs ont un pouvoir hydrophile de 1100% en poids (produit A) et de 500% en poids (produit Abis), et que le produit de référence B a un pouvoir hydrophile de 400 à 600% (en moyenne 500%), selon la technique décrite ci-dessus.

Le polymère selon l'invention est stable à la chaleur. Il n'est pas dégradé à une température pouvant aller jusqu'à 200°C ou plus. En particulier, il n'est pas dégradé par une exposition à 150°C pendant 1 h. Il est stérilisable à la chaleur, par UV ainsi qu'à la vapeur d'oxyde d'éthylène.

Le polymère selon l'invention peut être présenté sous forme de gel, de poudre, de billes ou de cristaux.

Le polymère selon l'invention, broyé dans l'eau, donne un gel particulaire possédant des propriétés thixotropiques. Sous cette forme, il peut être utilisé comme agent hydratant de la peau. Le gel peut également être utilisé en couche mince comme base de maquillage et atténuateur de rides. Ce gel peut encore être utilisé dans le domaine des pansements, dans le cas d'enflures à caractère inflammatoire, ou quand on veut diminuer par exemple la température corporelle locale (par exemple en enroulant le pansement comprenant ledit gel autour des mollets).

Le polymère sous forme de poudre, de billes ou de cristaux peut être utilisé directement sur les plaies, peut être administré par voie orale (agent séquestrant des acides biliaires et/ou agent anorexigène), ou encore utilisé comme substance échangeuse d'ions.

Le meilleur mode de mise en œuvre de l'invention a été décrit ci-après.

Au stade 1), on condense un polyoléther I (qui a été préalablement déshydraté à 120°C sous vide pendant 3 h et qui a un poids moléculaire équivalent de 100 à 160 et un indice OH de 475 à 505) avec un polyisocyanate IV choisi parmi l'ensemble constitué par le 2,4-toluènediisocyanate et le toluènediisocyanate commercial renfermant 80% en poids d'isomère-2,4 et 20% en poids d'isomère-2,6, à une température de 100–140°C, pendant 2–3 h, sous atmosphère d'azote, en présence d'un excès de groupes OH libres provenant de I par rapport aux groupes NCO libres provenant de IV, la réaction de condensation étant effectuée de telle façon que le rapport numérique du nombre de groupes NCO libres provenant de IV au nombre de groupes OH libres provenant de I soit compris entre (1:3) et (3:4). Parmi les polyuréthane-polyols (1:3)–(3:4), les produits les plus intéressants sont les polyuréthane-polyols (1:2)–(2:3).

On gonfle ensuite le polyuréthane-polyol V ainsi obtenu au moyen de diméthylformamide, de telle façon que le polymère ait un volume de 2 à 20 fois, de préférence 5 à 10 fois, son volume initial. Après le gonflement, on broie le polyuréthane-polyol, puis sèche à 50°C et élimine le diméthylformamide.

Au stade 2), on fait réagir dans un solvant (eau ou dichloroéthane) le polyuréthane-polyol V (1 à 20 parties en poids) avec l'épichlorhydrine (30 à 60 parties en poids) et la polyamine (40 à 100 parties en poids) – la polyamine préférée étant l'hexaméthylènediamine, les quantités préférées de polyuréthane-polyol–épichlorhydrine–hexaméthylènediamine étant de (1:3:6) parties en poids à (1:6,6:10) parties en poids –, à une température de 60–70°C, sous agitation, jusqu'à ce que le milieu réactionnel se prenne en masse (20–60 min), puis, après prise en masse, à une température de 60–70°C jusqu'à ce que l'épichlorhydrine et la polyamine aient été éliminées sans qu'il soit nécessaire d'agiter, la polyamine étant utilisée sous forme de base libre ou sous forme de chlorhydrate (notamment obtenu par salification de la base libre avec HCl 10N, jusqu'à pH 1–3). On recueille le polymère attendu par essorage (sous aspiration), séchage à l'étuve à 50°C pendant 48 h, avec un rendement compris entre 60 et 80%.

Le produit obtenu selon le meilleur mode de mise en œuvre à partir d'un polyuréthane-polyol (1:2) d'épichlorhydrine et d'hexaméthylènediamine, qui a pour numéro de code HP 102, est une substance cristalline fondant à 240°C environ et ayant une densité de 1,1 g/cm³. Cette substance peut, après usage, être régénérée par essorage et séchage à l'étuve, comme indiqué ci-dessus, puis stérilisée.

D'autres avantages et caractéristiques de l'invention seront mieux compris à la lecture qui va suivre des exemples de préparation.

Exemple 1

1. On déshydrate à 120°C sous vide pendant 3 h 100 g de polyolpolyéther (ayant un poids moléculaire équivalent de 100 à 160 et un indice OH de 475 à 505 et résultant de la réaction de condensation du sorbitol avec l'oxyde de propylène), puis on fait réagir 40 g de toluènediisocyanate (mélange de 80% en poids d'isomère 2,4 et de 20% en poids d'isomère 2,6) entre 100 et 140°C pendant 1 à 3 h. On obtient 130 g de polyuréthane-polyol (1:2) solide qu'on laisse reposer pendant 12 h à 15–25°C. On gonfle ensuite ce polyuréthane au moyen de diméthylformamide jusqu'à 10 fois son volume, puis on le broie en particules les plus fines possible. On sèche ces particules, les lave à l'eau, filtre, contracte dans le volume suffisant d'alcool (CH₃OH ou C₂H₅OH), filtre à nouveau, contracte dans l'eau, puis lave à l'eau jusqu'à ce que l'alcool et le solvant gonflant (DMF) soient éliminés.

2. On fait réagir dans de l'eau, à 70°C, 50 g de polyuréthane ainsi traité avec 200 g d'épichlorhydrine, puis 300 g d'hexaméthylènediamine, la durée de prise étant de 20 min, la réaction étant poursuivie après la prise en masse pendant 4 h à 70°C. On obtient un gel consistant qui devient cristallin sous agitation à l'état gonflé. On essore puis sèche à 50°C pendant 48 h dans une étuve, à pression atmosphérique.

Pouvoir absorbant (en moyenne):
Eau: 2800% (28 fois son poids d'eau)
Plasma: 1800% (18 fois son poids de plasma)
Densité: 1,1 g/cm³
Point de fusion: 240°C
Rendement: 60%

Exemple 2

On gonfle 50 g de polyuréthane-polyol (1:2) obtenu selon le stade 1 de l'exemple 1, dans un minimum d'eau distillée, on ajoute ensuite 300 g d'hexaméthylènediamine dissous dans 200 ml d'eau distillée. Le mélange est agité et est chauffé à 70°C, après 10 min, on ajoute 200 g d'épichlorhydrine. La prise en masse intervient au bout de 1 h, on arrête alors l'agitation et on laisse chauffer encore pendant 4 h, on ajoute ensuite HCl 10N jusqu'à pH 2–3. Le produit est ensuite lavé dans l'eau, le gel obtenu s'agglomère et les particules adhèrent faiblement les unes aux autres. Le produit est contracté avec de l'éthanol à 96%, essoré et séché à l'étuve sous pression atmosphérique pendant 48 h à 50°C.

Rendement: 60%
Pouvoir hydrophile: 18 à 24 fois son poids (20 fois en moyenne)

Exemple 3

On mélange dans un réacteur, muni d'un agitateur, 50 g de polyuréthane-polyol (1:2) préparé selon le stade 1) de l'exemple 1, et 100 ml de dichloroéthane, on ajoute 300 g d'hexaméthylènediamine, puis on introduit goutte à goutte, pendant 20 min à 70°C, 200 g d'épichlorhydrine; le milieu réactionnel étant pris en masse, on poursuit l'agitation pendant 8 h. Le mélange est ensuite acidifié avec de l'acide chlorhydrique 10N, jusqu'à pH 1. Le produit est lavé à l'eau distillée jusqu'à pH 7. Après contraction avec de l'éthanol, le produit obtenu est séché à l'étuve, à 50°C pendant 48 h.

Rendement: 80%
Pouvoir hydrophile: 26 à 32 fois son poids (28 fois en moyenne)

Exemple 4

Dans un récipient de réaction, muni d'un agitateur, on mélange 50 g de polyuréthane-polyol (1:2) préparé selon le stade 1) de l'exemple 1 avec le minimum d'eau distillée, et on introduit 150 g d'épichlorhydrine. On ajoute ensuite 300 g d'hexaméthylènediamine tout en agitant à 70°C.

La prise en masse intervient au bout de 20 min.

La réaction est poursuivie pendant 4 h. Le mélange est ensuite acidifié à pH 1 avec HCl 10N. Le produit est lavé dans l'eau jusqu'à pH 7. Après contraction dans l'éthanol, le produit est séché à 50°C pendant 48 h.

Rendement: 60%
Pouvoir hydrophile: 18 à 24 fois en poids (20 fois en moyenne)

Exemple 5

(numéro de code HP 102)

1. On condense à 100–140°C, pendant 2 h, sous agitation et sous atmosphère d'azote, 100 g de polyolpolyéther (préalablement séché à 120°C sous vide pendant 3 h, ayant un poids moléculaire moyen de 702, un poids moléculaire équivalent de 117 et un indice OH de 475–505, préparé à partir de sorbitol et d'oxyde de propylène et commercialisé sous le nom de «UGIPOL 3540») avec 37 g de toluènediisocyanate (ayant un poids moléculaire de 174 et un poids moléculaire équivalent de 87, et commercialisé sous le nom de «LILENE T 80»). Le polyuréthane-polyol (1:2) ainsi obtenu est gonflé dans le diméthylformamide jusqu'à dix fois son volume initial, puis est broyé, lavé à l'eau, contracté dans le volume minimal de $C_2H_5OH$, filtré, contracté dans l'eau, puis lavé à l'eau.

2. On fait réagir dans de l'eau 30 g de polyuréthane-polyol (1:2) ainsi obtenu, avec 200 g d'épichlorhydrine et 300 g d'hexaméthylènediamine, cette dernière substance étant introduite goutte à goutte dans le milieu réactionnel constitué par les autres ingrédients. La réaction est menée à 70°C pendant 40 min sous agitation, jusqu'à ce qu'il y ait prise en masse, puis est poursuivie sous agitation pendant 4 h à 70°C. On ajoute HCl 10N jusqu'à pH 2–3, lave à l'eau jusqu'à pH 7, essore, puis sèche à 50°C pendant 48 h sous pression atmosphérique.

Rendement: 80%
Pouvoir hydrophile: 26 à 32 fois son poids (28 fois en moyenne)
Point de fusion: 240°C

Exemple 5bis

En procédant comme indiqué à l'exemple 5 et en remplaçant les proportions respectives de polyuréthane-polyol (1:2)-épichlorhydrine-hexaméthylènediamine qui sont de (1:6,6:10) parties en poids, par les proportions de (1:6,6:9) parties en poids, on obtient le même polymère avec un rendement de 80%.

L'exemple 6 qui suit illustre l'obtention d'un polymère selon l'invention, à partir d'une faible quantité de polyuréthane-polyol par rapport aux quantités d'épichlorhydrine et d'hexaméthylènediamine.

Exemple 6

On dissout dans 800 ml d'eau distillée tout en agitant à 70°C 5 g de polyuréthane-polyol (1:2) préparé selon le stade 1) de l'exemple 5, et 180 g d'épichlorhydrine. Après 10 min, on ajoute 220 g d'hexaméthylènediamine dans 50 ml d'eau. La durée de prise est de 40 min; la réaction est poursuivie à 70°C pendant 4 h. L'acidification à pH 1 est faite avec HCl 10N. Le produit est délayé deux fois dans l'eau et contracté avec de l'éthanol; ensuite on sèche le produit à 50°C pendant 48 h à l'étuve sous pression atmosphérique.

Rendement: 60%
Pouvoir hydrophile: 16 à 18 fois son poids (17 fois en moyenne)

L'exemple 7 qui suit illustre l'obtention d'un polymère selon l'invention à partir d'une quantité importante de polyuréthane-polyol par rapport à celles de l'épichlorhydrine et de l'hexaméthylène-diamine.

Exemple 7

Dans un réacteur muni d'un agitateur, on mélange 100 g de polyuréthane-polyol (1:2), préparé selon le stade 1) de l'exemple 5, dans une quantité minimale d'eau distillée avec 180 g d'épichlorhydrine, on ajoute ensuite 270 g d'hexaméthylènediamine dans 500 ml d'eau. Le mélange est agité et est chauffé à 70°C pendant 1 h jusqu'à prise en masse. La réaction est poursuivie pendant 4 h à 70°C, on ajoute 10 ml d'acide chlorhydrique 10N dans le mélange. Le produit est délayé deux fois dans l'eau, il est contracté dans l'éthanol et séché à 50°C pendant 48 h.

Rendement: 60%
Pouvoir hydrophile: 18–22 fois son poids (20 fois en moyenne)

Exemple 8

On mélange 30 g de polyuréthane-polyol (1:2), préparé selon le stade 1) de l'exemple 5, 180 g d'épichlorhydrine dans 800 ml d'eau distillée; après 10 min d'agitation, on ajoute 200 g d'hexaméthylènediamine dissous dans 500 ml d'eau. La prise en masse intervient au bout de 40 min à 70°C, sous agitation. La réaction est poursuivie pendant 4 h à 70°C. Le produit est acidifié à pH 1 avec l'acide chlorhydrique 10N, lavé dans l'eau, contracté avec de l'éthanol et séché à l'étuve à 50°C pendant 48 h.

Rendement: 60%
Pouvoir hydrophile: 24 à 30 fois son poids (26 fois en moyenne)

Exemple 9

Dans un réacteur muni d'un agitateur, on mélange 30 g de polyuréthane-polyol (2:3), préparé comme indiqué à l'exemple 5 à partir du polyolpolyéther et du toluènediisocyanate à raison de 2 groupes NCO pour 3 groupes OH, dans 800 ml d'eau et 180 g d'épichlorhydrine. Après 10 min d'agitation, on ajoute 200 g d'hexaméthylènediamine dissous dans 500ml d'eau. La prise en masse intervient au bout de 40 min à 70°C, sous agitation. La réaction est poursuivie pendant 4 h à 70°C. Le produit est acidifié, lavé, contracté dans l'éthanol et séché à 50°C pendant 48 h.

Rendement: 60%
Pouvoir hydrophile: 22 à 28 fois son poids (26 fois en moyenne)

Exemple 10

Dans un ballon muni d'un agitateur, on mélange 30 g de polyuréthane-polyol (2:3) obtenu comme indiqué à l'exemple 9 et gonflé dans 500 ml de dichloro-éthane, et 180 g d'épichlorhydrine. Après 10 min d'agitation à 60°C, on ajoute 200 g d'hexaméthylènediamine dissous dans 500 ml d'eau. La prise en masse intervient au bout de 20 min. La réaction est ensuite poursuivie pendant 8 h à 60°C. Le produit obtenu est acidifié, lavé, contracté et séché à 50°C pendant 48 h.

Rendement: 80%
Pouvoir hydrophile: 20 à 26 fois son poids (24 fois en moyenne)

Exemple A (comparatif)

On mélange 270 g d'hexaméthylènediamine et 600 ml d'eau dans un réacteur muni d'un agitateur. On ajoute goutte à goutte 200 g d'épichlorhydrine dans 500 ml de dichloroéthane. On chauffe à 60°C tout en agitant pendant 20 min, puis sans agitation pendant 8 h. Le gel formé est acidifié avec de l'acide chlorhydrique 10N jusqu'à pH 1. Le produit est lavé dans l'eau distillée jusqu'à pH 7, contracté avec de l'éthanol à 96% et séché à l'étuve à 50°C pendant 48 h.

Rendement: 80%
Pouvoir hydrophile: 10 à 12 fois son poids (11 fois en moyenne)

Exemple Abis (comparatif)

Dans un ballon muni d'un agitateur, on mélange 30 g de polyuréthane-polyol (1:2), préparé selon le stade 1) de l'exemple 1, et gonflé dans 500 ml de dichloroéthane, et 180 g d'épichlorhydrine. Après 10 min d'agitation à 60°C, on ajoute 200 g de carbailide dissous dans 500 ml d'eau. La prise en masse intervient au bout de 60 min, puis la réaction est poursuivie pendant 4 h à 60°C. Le produit obtenu est acidifié, lavé, contracté et séché à 50°C pendant 48 h.

Rendement: 25%
Pouvoir hydrophile: 4 à 6 fois son poids (5 fois en moyenne)

Exemple Ater

Quand on fait réagir le polyuréthane-polyol V avec l'hexaméthylènediamine dans de l'eau à 60-70°C, sous agitation (sans utiliser d'épichlorhydrine), on obtient un élastomère peu hydrophile mais collant.

Exemple 11

On prépare un polyuréthane-polyol (1:3) selon le procédé du stade 1 de l'exemple 1 par déshydratation à 120°C sous vide pendant 3 h de 100 g du polyol-polyéther puis réaction avec 25 g de toluènediisocyanate. On procède ensuite à l'introduction de l'épichlorhydrine et de l'hexaméthylènediamine selon le procédé décrit au stade 2 de l'exemple 1.

Exemple 12

On prépare un polyuréthane-polyol (3:4) selon le procédé du stade 1 de l'exemple 1 à partir de 100 g du polyol-polyéther et de 60 g de toluènediisocyanate. On procède ensuite à l'introduction de l'épichlorhydrine et de l'hexaméthylènediamine selon le procédé du stade 2 de l'exemple 1.

Les modalités opératoires du stade 2) pour l'obtention des polymères des exemples précédents sont résumées dans le tableau I ci-après avec les pouvoirs gonflants desdits exemples.

Le polymère obtenu selon le procédé de l'invention, et plus précisément celui des exemples 1–12, est sous forme de poudre à l'état cristallisé, les particules ayant un diamètre compris entre 5 et 50 µm (en moyenne 10 µm).

Les essais pharmacologiques et cliniques qui ont été entrepris ont mis en évidence que le polymère selon l'invention est peu toxique, notamment par voie orale, et qu'il est utile en thérapeutique (traitement des plaies pour absorber les liquides corporels, séquestration des acides biliaires, action anorexigène du fait de son gonflement dans l'estomac) et en cosmétique.

Tableau I

| Exemple | 1 | 2 | 3 | 4 | 5 | 6 |
|---|---|---|---|---|---|---|
| Polyuréthane-polyol V | (1:2) | (1:2) | (1:2) | (1:2) | (1:2) | (1:2) |
| Solvant | eau | eau | dichloro-éthane | eau | eau | eau |
| Mode d'introduction des réactifs | (a) | (b) | (b) | (a) | (a) | (a) |
| Quantités respectives (d) | 1:4:6 | 1:4:6 | 1:4:6 | 1:3:6 | 1:6, 6:10 | 1:36:44 |
| Durée de réaction — prise | 20 min | 60 min | 20 min | 40 min | 40 min | 40 min |
| Durée de réaction — après prise | 4 h | 4 h | 8 h | 4 h | 4 h | 4 h |
| Température de réaction | 70°C | 70°C | 60°C | 70°C | 70°C | 70°C |
| Séchage — durée | 48 h | 48 h | 48 h | 48 h | 48 h | 48 h |
| Séchage — température | 50°C | 50°C | 50°C | 50°C | 50°C | 50°C |
| Rendement | 60% | 60% | 80% | 60% | 80% | 60% |
| Pouvoir hydrophile (e) | 26p–32p | 18p–24p | 26p–32p | 18p–24p | 26p–32p | 16p–18p |
| (moyenne) | (28p) | (20p) | (28p) | (20p) | (28p) | (17p) |

Tableau I (suite)

| Exemple | 7 | 8 | 9 | 10 | A | Abis |
|---|---|---|---|---|---|---|
| Polyuréthane-polyol V | (1:2) | (1:2) | (2:3) | (2:3) | – | (1:2) |
| Solvant | eau | eau | eau | dichloroéthane | dichloroéthane | dichloroéthane |
| Mode d'introduction des réactifs | (a) | (a) | (a) | (a) | (b) | (c) |
| Quantités respectives (d) | 1:1,8:2,7 | 1:6:6,6 | 1:6:6,6 | 1:6:6,6 | VI–VII=1:1,35 | 1:6:6,6 |
| Durée de prise | 60 min | 40 min | 40 min | 20 min | 20 min | 60 min |
| réaction après prise | 4 h | 4 h | 4 h | 8 h | 8 h | 4 h |
| Température de réaction | 70°C | 70°C | 70°C | 60°C | 60°C | 60°C |
| Séchage durée | 48 h | 48 h | 48 h | 48 h | 48 h | 48 h |
| témpérature | 50°C | 50°C | 50°C | 50°C | 50°C | 50°C |
| Rendement | 60% | 60% | 60% | 80% | 80% | 25% |
| Pouvoir hydrophile (e) | 18p–22p | 24p–30p | 22p–28p | 20p–26p | 10p–12p | 4p–6p |
| (moyenne) | (20p) | (28p) | (26p) | (24p) | (11p) | (5p) |

Notes: (a): épichlorhydrine avant hexaméthylènediamine;
      (b): hexaméthylènediamine avant épichlorhydrine;
      (c): épichlorhydrine avant carbailide ($C_6H_5NHCONHC_6H_5$);
      (d): quantités respectives en parties en poids de
          – polyuréthane-polyol-épichlorhydrine-hexaméthylènediamine pour exemples 1–10,
          – épichlorhydrine-hexaméthylènediamine pour exemple A, et
          – polyuréthane-polyol-épichlorhydrine-carbailide pour exemple Abis;
      (e): exprimé en poids par rapport au poids p du polymère obtenu après séchage du stade 2).

Le polymère selon l'invention sous forme de poudre (notamment sous forme de particules sphériques ou cirstallines) peut être projeté simultanément avec des fibres sur un support transfert (papier siliconé) ou sur un support définitif (tulle gras, biogaze ou crêpe). L'ensemble obtenu est un produit composite, les particules de polymère et les fibres constituant un non-tissé qui est utilisable dans le domaine des pansements, ledit non-tissé facilitant l'application et le retrait du polymère de la plaie.

On a représenté sur la fig. 3 le mode d'obtention schématisé du non-tissé constitué par le polymère selon l'invention et les fibres. Le polymère en poudre 7, qui provient d'une trémie 11 comprenant une vis 12 de distribution entraînée par un moteur 20, donne un flux 18 entraîné vers le support 10 (de préférence un support transfert en papier siliconé) par un flux 17 provenant d'un dispositif de projection 13 entraîné par un moteur 16 et recevant de l'air comprimé 14 et un élastomère [copolymère butadiène-styrène (50:50) en poids] en solution dans un solvant (notamment acétate d'éthyle) qui est introduit en 15. Lors de la projection au moyen de l'air comprimé 14, l'élastomère est séché et donne des fibres véhiculées par le flux 17.

L'ensemble des flux 17 et 18 permet le dépôt en 19 sur le support 10, au fur et à mesure du déplacement de ce dernier, de fibres et de polymère selon l'invention.

On peut par ailleurs conditionner le polymère en poudre selon l'invention dans un sachet antiadhésif et poreux (notamment tissé ou non-tissé) pour la réalisation de pansements.

On peut également utiliser le polymère en poudre selon l'invention en tant qu'agent gonflant pour l'incorporer dans les couches pour bébés et les alèses pour vieillards et sujets incontinents. Dans ce type d'application, l'agent gonflant peut être conditionné à l'état sec dans des poches perméables aux liquides disposées dans lesdites couches et alèses.

On peut également utiliser le polymère en poudre selon l'invention à l'état gonflé (avec de l'eau) et conditionné dans une ou plusieurs poches poreuses en tant que saturateur, notamment pour radiateurs.

On a enfin observé que le polymère selon l'invention présente des propriétés antiseptiques bénéfiques en thérapeutique et en cosmétique.

Lorsque la composition est administrée en thérapeutique par voie locale sous forme de pansement pour le traitement des inflammations, le polymère gonflé qu'elle renferme agit en tant qu'agent anti-inflammatoire.

**Revendications pour les états contractants: BE, CH, LI, DE, FR, GB, IT, LU, NL, SE**

1. Procédé de préparation d'un polymère de structure tridimensionnelle du type polyuréthane réticulé et ayant un bon pouvoir gonflant, ledit procédé étant caractérisé en ce que
   1) on fait réagir
   a) un polyoléther, choisi parmi l'ensemble constitué par les polyols oxyalkylénés (I) et leurs mélanges, obtenu par condensation d'un polyol (II) renfermant au moins deux groupes OH avec un oxyde d'alkylène (III) à raison de 1 à 20 moles de (III) par groupe OH libre de (II), avec
   b) un polyisocyanate (IV) renfermant au moins deux groupes NCO libres par molécule,
   la réaction étant effectuée avec un excès de groupes OH de (I) par rapport aux groupes isocyanates NCO de (IV) pour obtenir un polyuréthane-polyol (V) ayant des groupes OH libres; et,

2) on fait réagir le polyuréthane-polyol (V) ainsi obtenu avec l'épichlorhydrine (VI) et une polyamine (VII) choisie parmi les polyamines possédant au moins deux groupes amino $NH_2$ et leurs mélanges.

2. Procédé selon la revendication 1, caractérisé en ce que le polyoléther du stade 1) est obtenu à partir d'un polyol II renfermant 3 à 6 groupes OH.

3. Procédé selon la revendication 1, caractérisé en ce que le polyoléther du stade 1) est obtenu à partir d'un polyol choisi parmi l'ensemble comprenant les alkylèneglycols, les triols, tétraols, pentols, hexols et leurs mélanges.

4. Procédé selon la revendication 3, caractérisé en ce que le polyoléther est obtenu à partir d'un polyol choisi parmi l'ensemble constitué par le sorbitol, le dulcitol, le pentaérythritol et leurs mélanges.

5. Procédé selon la revendication 1, caractérisé en ce que le polyisocyanate utilisé au stade 1) est choisi parmi l'ensemble constitué par les 2,4-toluènediisocyanate, 2,6-toluènediisocyanate, diphényl-méthyldiisocyanate et leurs mélanges.

6. Procédé selon la revendication 5, caractérisé en ce que le polyisocyanate est choisi parmi le 2,4-toluènediisocyanate et le mélange pondéral 2,4-toluènediisocyanate-2,6-toluènediisocyanate (80:20).

7. Procédé selon la revendication 1, caractérisé en ce que la réaction du stade 1) est effectuée à une température comprise entre 20 et 150°C pendant au moins 1 heure sous atmosphère d'azote.

8. Procédé selon la revendication 7, caractérisé en ce que la réaction du stade 1) du polyoléther avec le polyisocyanate est effectuée à une température comprise entre 100 et 140°C pendant deux à trois heures.

9. Procédé selon l'une quelconque des revendications 1, 7 et 8, caractérisé en ce que la réaction du stade 1) est effectuée avec des quantités respectives du polyoléther I et de polyisocyanate IV telles que le rapport numérique du nombre de groupes NCO libres provenant de IV au nombre de groupes OH libres provenant de I soit compris entre (1:3) et (3:4).

10. Procédé selon la revendication 9, caractérisé en ce que la réaction du stade 1) est effectuée avec des quantités respectives de polyoléther I et de polyisocyanate IV telles que le rapport numérique nombre de groupes NCO libres provenant de IV/nombre de groupes OH libres provenant de I, soit compris entre (1:2) et (2:3).

11. Procédé selon la revendication 1, caractérisé en ce qu'après le stade 1) mais avant le stade 2), le polyuréthane-polyol V obtenu est soumis à un gonflement au moyen d'une substance choisie parmi l'ensemble constitué par l'eau, les solvants organiques et leurs mélanges.

12. Procédé selon la revendication 11, caractérisé en ce que le moyen de gonflement est le diméthylformamide.

13. Procédé selon l'une quelconque des revendications 11 et 12, caractérisé en ce que le gonflement est mis en œuvre de telle façon que le polyuréthane-polyol V ait un volume de 2 à 20 fois son volume initial.

14. Procédé selon la revendication 13, caractérisé en ce que le gonflement est mis en œuvre de telle façon que le polyuréthane-polyol V ait un volume de 5 à 10 fois son volume initial.

15. Procédé selon la revendication 1, caractérisé en ce que la réaction du stade 2) est (i) effectuée à une température comprise entre 20 et 100°C pendant 2 minutes à 2 h, sous agitation, jusqu'à ce que le milieu réactionnel se gélifie, puis (ii) poursuivie, l'agitation n'étant plus nécessaire, à une température de 60–70°C jusqu'à ce que les composés (VI) et (VII) aient disparu du milieu réactionnel.

16. Procédé selon l'une quelconque des revendications 1 et 15, caractérisé en ce que la polyamine du stade 2) est choisie notamment parmi les diamines aliphatiques, aromatiques et aralkyliques.

17. Procédé selon la revendication 16, caractérisé en ce que la polyamine du stade 2) est l'hexaméthylènediamine.

18. Procédé selon la revendication 1, caractérisé en ce que la réaction du stade 2) est effectuée en présence d'un solvant choisi parmi l'eau, l'acétone, le diméthylformamide, le tétrahydrofuranne, le dichloroéthane, le chloroforme, le tétrachlorure de carbone et leurs mélanges.

19. Procédé selon l'une quelconque des revendications 1 et 15 à 18, caractérisé en ce que le produit obtenu au stade 2) est soumis à un essorage sous vide ou à une aspiration puis séché à 40–80°C pendant 24 à 72 h.

20. Procédé selon la revendication 1, caractérisé en ce que:

– on condense un polyoléther I (qui a été préalablement déshydraté à 120°C sous vide pendant 3 h et qui a un poids moléculaire équivalent de 100 à 160 et un indice OH de 475 à 505) avec un polyisocyanate IV choisi parmi l'ensemble constitué par le 2,4-toluènediisocyanate et le toluènediisocyanate commercial renfermant 80% en poids d'isomère 2,4 et 20% en poids d'isomère 2,6, à une température de 100–140°C pendant 2–3 h, sous atmosphère d'azote, en présence d'un excès de groupes OH libres provenant de I par rapport aux groupes NCO libres provenant de IV, la réaction de condensation étant effectuée de telle façon que le rapport numérique du nombre de groupes NCO libres provenant de IV au nombre de groupes OH libres provenant de I soit compris entre (1:3) et (3:4);

– on gonfle ensuite le polyuréthane-polyol V ainsi obtenu, au moyen de diméthylformamide de telle façon que le polymère ait un volume de 5 à 10 fois son volume initial; puis on broie le polyuréthane-polyol, sèche à 50°C et élimine le diméthylformamide;

– on fait réagir dans un solvant choisi parmi l'eau et le dichloroéthane, 1 à 20 parties en poids de polyuréthane-polyol V avec 30 à 60 parties en poids d'épichlorhydrine et 40 à 100 parties en poids d'hexaméthylènediamine, à une température de 60-70°C sous agitation pendant 20 à 60 minutes jusqu'à ce que le milieu réactionnel se prenne en masse, puis à une température de 60-70°C jusqu'à ce que l'épichlorhydrine et l'hexaméthylène-diamine aient disparu du milieu réactionnel, l'hexaméthylènediamine étant utilisée sous forme de base libre ou de chlorhydrate; et

   – on recueille le polymère attendu par essorage puis séchage à 50°C pendant 48 heures.

21. Procédé selon la revendication 20, caractérisé en ce que les quantités de polyuréthanepolyol-épichlorhydrine-hexaméthylènediamine sont dans le rapport pondéral (1:3:6) à (1:6,6:10).

22. Polymère de structure tridimensionnelle du type polyuréthane réticulé et ayant un bon pouvoir gonflant, caractérisé en ce qu'il est préparé selon le procédé de l'une quelconque des revendications 1 à 21.

23. Polymère de structure tridimensionnelle selon la revendication 22, caractérisé en ce qu'il a une densité de 1,1 g/cm³, en ce qu'il est cristallin et fond à 240°C.

24. Procédé selon l'une quelconque des revendications 22 et 23, caractérisé en ce que son pouvoir gonflant est vis-à-vis de l'eau, de 1500 à 4000% en poids, et vis-à-vis du plasma de 1200 à 2500% en poids.

25. Polymère selon la revendication 24, caractérisé en ce qu'il a été obtenu à partir de une partie en poids de polyuréthane polyol V, 4 parties en poids d'épichlorhydrine et 6 parties en poids d'hexaméthylènediamine, le polyuréthanepolyol V ayant été obtenu par réaction d'un polyoléther I avec un polyisocyanate IV de façon que le rapport numérique du nombre de groupes NCO libres provenant de IV au nombre de groupes OH libres provenant de I soit de (1:2).

26. Composition utile notamment en thérapeutique et en cosmétique, caractérisée en ce qu'elle renferme un polymère selon l'une quelconque des revendications 22 à 25 en tant qu'ingrédient actif.

27. Composition selon la revendication 26, caractérisée en ce que le polymère qu'elle renferme en tant qu'ingrédient actif agit en tant qu'agent gonflant.

28. Composition selon la revendication 26, caractérisée en ce que le polymère qu'elle renferme en tant qu'ingrédient actif est gonflé au moyen d'eau.

29. Composition thérapeutique selon la revendication 27, caractérisée en ce qu'elle est administrée par voie orale et en ce que le polymère gonflant qu'elle renferme agit en tant qu'agent anorexigène et sequestrant des acides biliaires.

30. Composition thérapeutique selon la revendication 27, caractérisée en ce qu'elle est administrée par voie locale, notamment sous forme de pansement pour le traitement des plaies, et en ce que le polymère gonflant qu'elle renferme est à l'état sec avant administration.

31. Composition cosmétique selon la revendication 28, caractérisée en ce que le polymère gonflé au moyen d'eau qu'elle renferme agit en tant qu'agent hydratant.

32. Composition thérapeutique selon la revendication 28, caractérisée en ce qu'elle est administrée par voie locale sous forme de pansement pour le traitement des inflammations, et en ce que le polymère gonflé qu'elle renferme agit en tant qu'agent anti-inflammatoire.

### Revendications pour l'état contractant: AT

1. Procédé de préparation d'un polymère de structure tridimensionnelle du type polyuréthane réticulé et ayant un bon pouvoir gonflant, ledit procédé étant caractérisé en ce que
   1) on fait réagir
   a) un polyoléther, choisi parmi l'ensemble constitué par les polyols oxyalkylénés (I) et leurs mélanges, obtenu par condensation d'un polyol (II) renfermant au moins deux groupes OH avec un oxyde d'alkylène (III) à raison de 1 à 20 moles de (III) par groupe OH libre de (II), avec
   b) un polyisocyanate (IV) renfermant au moins deux groupes NCO libres par molécule,
   la réaction étant effectuée avec un excès de groupes OH de (I) par rapport aux groupes isocyanates NCO de (IV) pour obtenir un polyuréthane-polyol (V) ayant des groupes OH libres; et,
   2) on fait réagir le polyuréthane-polyol (V) ainsi obtenu avec l'épichlorhydrine (VI) et une polyamine (VII) choisie parmi les polyamines possédant au moins deux groupes amino $NH_2$ et leurs mélanges.

2. Procédé selon la revendication 1, caractérisé en ce que le polyoléther du stade 1) est obtenu à partir d'un polyol II renfermant 3 à 6 groupes OH.

3. Procédé selon la revendication 1, caractérisé en ce que le polyoléther du stade 1) est obtenu à partir d'un polyol choisi parmi l'ensemble comprenant les alkylèneglycols, les triols, tétraols, pentols, hexols et leurs mélanges.

4. Procédé selon la revendication 3, caractérisé en ce que le polyoléther est obtenu à partir d'un polyol choisi parmi l'ensemble constitué par le sorbitol, le dulcitol, le pentaérythritol et leurs mélanges.

5. Procédé selon la revendication 1, caractérisé en ce que le polyisocyanate utilisé au stade 1) est choisi parmi l'ensemble constitué par les 2,4-toluènediisocyanate, 2,6-toluènediisocyanate, diphényl-méthyldiisocyanate et leurs mélanges.

6. Procédé selon la revendication 5, caractérisé en ce que le polyisocyanate est choisi parmi le 2,4-toluènediisocyanate et le mélange pondéral 2,4-toluènediisocyanate-2,6-toluènediisocyanate (80:20).

7. Procédé selon la revendication 1, caractérisé en ce que la réaction du stade 1) est effectuée à une température comprise entre 20 et 150°C pendant au moins 1 heure sous atmosphère d'azote.

8. Procédé selon la revendication 7, caractérisé en ce que la réaction du stade 1) du polyoléther avec le polyisocyanate est effectuée à une température comprise entre 100 et 140°C pendant deux à trois heures.

9. Procédé selon l'une quelconque des revendications 1, 7 et 8, caractérisé en ce que la réaction du stade 1) est effectuée avec des quantités respectives du polyoléther I et de polyisocyanate IV telles que le rapport numérique du nombre de groupes NCO libres provenant de IV au nombre de groupes OH libres provenant de I soit compris entre (1:3) et (3:4).

10. Procédé selon la revendication 9, caractérisé en ce que la réaction du stade 1) est effectuée avec des quantités respectives de polyoléther I et de polyisocyanate IV telles que le rapport numérique nombre de groupes NCO libres provenant de IV/nombre de groupes OH libres provenant de I, soit compris entre (1:2) et (2:3).

11. Procédé selon la revendication 1, caractérisé en ce que après le stade 1) mais avant le stade 2), le polyuréthane-polyol V obtenu est soumis à un gonflement au moyen d'une substance choisie parmi l'ensemble constitué par l'eau, les solvants organiques et leurs mélanges.

12. Procédé selon la revendication 11, caractérisé en ce que le moyen de gonflement est le diméthylformamide.

13. Procédé selon l'une quelconque des revendications 11 et 12, caractérisé en ce que le gonflement est mis en œuvre de telle façon que le polyuréthane-polyol V ait un volume de 2 à 20 fois son volume initial.

14. Procédé selon la revendication 13, caractérisé en ce que le gonflement est mis en œuvre de telle façon que le polyuréthane-polyol V ait un volume de 5 à 10 fois son volume initial.

15. Procédé selon la revendication 1, caractérisé en ce que la réaction du stade 2) est (i) effectuée à une température comprise entre 20 et 100°C pendant 2 minutes à 2 h, sous agitation, jusqu'à ce que le milieu réactionnel se gélifie, puis (ii) poursuivie, l'agitation n'étant plus nécessaire, à une température de 60–70°C jusqu'à ce que les composés (VI) et (VII) aient disparu du milieu réactionnel.

16. Procédé selon l'une quelconque des revendications 1 et 15, caractérisé en ce que la polyamine du stade 2) est choisie notamment parmi les diamines aliphatiques, aromatiques et aralkyliques.

17. Procédé selon la revendication 16, caractérisé en ce que la polyamine du stade 2) est l'hexaméthylènediamine.

18. Procédé selon la revendication 1, caractérisé en ce que la réaction du stade 2) est effectuée en présence d'un solvant choisi parmi l'eau, l'acétone, le diméthylformamide, le tétrahydrofuranne, le dichloroéthane, le chloroforme, le tétrachlorure de carbone et leurs mélanges.

19. Procédé selon l'une quelconque des revendications 1 et 15 à 18, caractérisé en ce que le produit obtenu au stade 2) est soumis à un essorage sous vide ou à une aspiration puis séché à 40–80°C pendant 24 à 72 h.

20. Procédé selon la revendication 1, caractérisé en ce que:

– on condense un polyoléther I (qui a été préalablement déshydraté à 120°C sous vide pendant 3 h et qui a un poids moléculaire équivalent de 100 à 160 et un indice OH de 475 à 505) avec un polyisocyanate IV choisi parmi l'ensemble constitué par le 2,4-toluènediisocyanate et le toluènediisocyanate commercial renfermant 80% en poids d'isomère 2,4 et 20% en poids d'isomère 2,6, à une température de 100–140°C pendant 2–3 h, sous atmosphère d'azote, en présence d'un excès de groupes OH libres provenant de I par rapport aux groupes NCO libres provenant de IV, la réaction de condensation étant effectuée de telle façon que le rapport numérique du nombre de groupes NCO libres provenant de IV au nombre de groupes OH libres provenant de I soit compris entre (1:3) et (3:4);

– on gonfle ensuite le polyuréthane-polyol V ainsi obtenu, au moyen de diméthylformamide de telle façon que le polymère ait un volume de 5 à 10 fois son volume initial; puis on broie le polyuréthane-polyol, sèche à 50°C et élimine le diméthylformamide;

– on fait réagir dans un solvant choisi parmi l'eau et le dichloroéthane, 1 à 20 parties en poids de polyuréthane-polyol V avec 30 à 60 parties en poids d'épichlorhydrine et 40 à 100 parties en poids d'hexaméthylènediamine, à une température de 60–70°C sous agitation pendant 20 à 60 minutes jusqu'à ce que le milieu réactionnel se prenne en masse, puis à une température de 60–70°C jusqu'à ce que l'épichlorhydrine et l'hexaméthylène-diamine aient disparu du milieu réactionnel, l'hexaméthylènediamine étant utilisée sous forme de base libre ou de chlorhydrate; et

– on recueille le polymère attendu par essorage puis séchage à 50°C pendant 48 heures.

21. Procédé selon la revendication 20, caractérisé en ce que les quantités de polyuréthanepolyol-épichlorhydrine-hexaméthylènediamine sont dans le rapport pondéral (1:3:6) à (1:6,6:10).

**Patentansprüche für die Vertragsstaaten: BE, CH, DE, FR, GB, IT, LI, LU, NL, SE**

1. Verfahren zur Herstellung eines Polymerisates mit dreidimensionaler Struktur vom Typ vernetztes Polyurethan mit gutem Quellvermögen, welches Verfahren dadurch gekennzeichnet ist, dass

1) a) ein Polyoläther, ausgewählt aus der Gruppe bestehend aus den Oxyalkylenpolyolen (I) und ihren Mischungen, erhalten durch Kondensation eines zumindest zwei OH-Gruppen enthaltenden Polyols (II) mit einem Alkylenoxid (III) in einem Verhältnis von 1 zu 20 Mol von (III) pro freie OH-Gruppe von (II), mit

b) einem zumindest zwei freie NCO-Gruppen pro Molekül enthaltenden Polyisocyanat (IV) zur Umsetzung gebracht wird, welche Reaktion mit einem Überschuss an OH-Gruppen von (I) in bezug auf die Isocyanatgruppen NCO von (IV) zur Gewinnung eines Polyurethan-Polyols (V) mit freien OH-Gruppen durchgeführt wird; und

2) das so erhaltene Polyurethan-Polyol (V) mit Epichlorhydrin (VI) und einem Polyamin (VII), ausgewählt aus den Polyaminen mit zumindest zwei Aminogruppen NH₂ und ihren Mischungen, zur Umsetzung gebracht wird.

2. Verfahren nach Anspruch 1, dadurch gekennzeichnet, dass der Polyoläther der Stufe 1) aus einem 3 bis 6 OH-Gruppen enthaltenden Polyol (II) erhalten wird.

3. Verfahren nach Anspruch 1, dadurch gekennzeichnet, dass der Polyoläther der Stufe 1) aus einem Polyol, ausgewählt aus der Gruppe umfassend Alkylenglykole, Triole, Tetraole, Pentole, Hexole und ihre Mischungen, erhalten wird.

4. Verfahren nach Anspruch 3, dadurch gekennzeichnet, dass der Polyoläther aus einem Polyol, ausgewählt aus der Gruppe bestehend aus Sorbit, Dulcit, Pentaerythrit und Mischungen davon, erhalten wird.

5. Verfahren nach Anspruch 1, dadurch gekennzeichnet, dass das in Stufe 1) verwendete Polyisocyanat ausgewählt ist aus der Gruppe bestehend aus 2,4-Toluoldiisocyanat, 2,6-Toluoldiisocyanat, Diphenyl-methyldiisocyanat und Mischungen davon.

6. Verfahren nach Anspruch 5, dadurch gekennzeichnet, dass das Polyisocyanat ausgewählt ist aus 2,4-Toluoldiisocyanat und der gewichtsmässigen Mischung 2,4-Toluoldiisocyanat:2,6-Toluoldiisocyanat (80:20).

7. Verfahren nach Anspruch 1, dadurch gekennzeichnet, dass die Reaktion der Stufe 1) bei einer Temperatur zwischen 20 und 150°C während zumindest 1 h unter Stickstoffatmosphäre durchgeführt wird.

8. Verfahren nach Anspruch 7, dadurch gekennzeichnet, dass die Reaktion der Stufe 1) des Polyoläthers mit Polyisocyanat bei einer Temperatur zwischen 100 und 140°C während zwei bis drei Stunden durchgeführt wird.

9. Verfahren nach einem der Ansprüche 1, 7 und 8, dadurch gekennzeichnet, dass die Reaktion der Stufe 1) mit solchen Mengen von Polyoläther (I) bzw. Polyisocyanat (IV) durchgeführt wird, dass das numerische Verhältnis der Anzahl an freien NCO-Gruppen von (IV) zur Anzahl an freien OH-Gruppen von (I) zwischen 1:3 und 3:4 beträgt.

10. Verfahren nach Anspruch 9, dadurch gekennzeichnet, dass die Reaktion der Stufe 1) mit solchen Mengen von Polyoläther (I) bzw. Polyisocyanat (IV) durchgeführt wird, dass das numerische Verhältnis der Anzahl von freien NCO-Gruppen von (IV)/Anzahl von freien OH-Gruppen von (I) zwischen 1:2 und 2:3 liegt.

11. Verfahren nach Anspruch 1, dadurch gekennzeichnet, dass nach der Stufe 1), aber vor der Stufe 2) das erhaltene Polyurethan-Polyol (V) einer Quellung mittels einer Substanz, ausgewählt aus der Gruppe Wasser, organische Lösungsmittel und Mischungen davon, unterzogen wird.

12. Verfahren nach Anspruch 11, dadurch gekennzeichnet, dass das Quellmittel Dimethylformamid ist.

13. Verfahren nach einem der Ansprüche 11 und 12, dadurch gekennzeichnet, dass die Quellung in einer Weise ausgeführt wird, dass das Polyurethan-Polyol (V) ein Volumen vom 2- bis 20fachen seines Ausgangsvolumens hat.

14. Verfahren nach Anspruch 13, dadurch gekennzeichnet, dass die Quellung in einer Weise ausgeführt wird, dass das Polyurethan-Polyol (V) ein Volumen vom 5 bis 10fachen seines Ausgangsvolumens hat.

15. Verfahren nach Anspruch 1, dadurch gekennzeichnet, dass die Reaktion der Stufe 2) (i) bei einer Temperatur zwischen 20 und 100°C während 2 min bis 2 h unter Rühren bis zum Gelieren der Reaktionsmischung und danach (ii) bei einer Temperatur von 60 bis 70°C, wobei ein Rühren nicht mehr notwendig ist, bis die Verbindungen (VI) und (VII) aus dem Reaktionsmedium verschwunden sind, durchgeführt wird.

16. Verfahren nach einem der Ansprüche 1 und 15, dadurch gekennzeichnet, dass das Polyamin der Stufe 2) insbesondere ausgewählt ist aus den aliphatischen, aromatischen und Aralkyl-Diaminen.

17. Verfahren nach Anspruch 16, dadurch gekennzeichnet, dass das Polyamin der Stufe 2) Hexamethylendiamin ist.

18. Verfahren nach Anspruch 1, dadurch gekennzeichnet, dass die Reaktion der Stufe 2) in Gegenwart eines Lösungsmittels, ausgewählt aus Wasser, Aceton, Dimethylformamid, Tetrahydrofuran, Dichloräthan, Chloroform, Tetrachlorkohlenstoff und Mischungen davon, durchgeführt wird.

19. Verfahren nach einem der Ansprüche 1 und 15 bis 18, dadurch gekennzeichnet, dass das in Stufe 2) erhaltene Produkt einem Lösungsmittelentzug unter Vakuum oder einer Absaugung unterworfen und danach während 24 bis 72 h bei 40 bis 80°C getrocknet wird.

20. Verfahren nach Anspruch 1, dadurch gekennzeichnet, dass

– ein Polyoläther (I) (der zuvor bei 120°C unter Vakuum während 3 h dehydratisiert wurde und ein Molgewicht von 100 bis 160 und einen OH-Index von 475 bis 505 aufweist) mit einem Polyisocyanat (IV), ausgewählt aus der Gruppe bestehend aus 2,4-Toluoldiisocyanat und kommerziellem Toluoldiisocyanat, enthaltend 80 Gew.-% 2,4-Isomeres und 20 Gew.-% 2,6-Isomeres, bei einer Temperatur von 100 bis 140°C während 2 bis 3 h unter Stickstoffatmosphäre in Gegenwart eines Überschusses an freien OH-Gruppen von (I) gegenüber freien NCO-Gruppen von (IV) kondensiert wird, wobei die Kondensationsreaktion derart durchgeführt wird, dass das numerische Verhältnis der Anzahl an freien NCO-Gruppen von (IV) zur Anzahl an freien OH-Gruppen von (I) zwischen 1:3 und 3:4 liegt;

– danach das so erhaltene Polyurethan-Polyol (V) mittels Dimethylformamid derart gequollen wird, dass das Polymerisat ein Volumen vom 5 bis 10fachen seines Ausgangsvolumens hat; anschliessend das Polyurethan-Polyol zerstossen wird, bei 50°C getrocknet wird und das Dimethylformamid entfernt wird;

– 1 bis 20 Gewichtsteile Polyurethan-Polyol (V) mit 30 bis 60 Gewichtsteilen Epichlorhydrin und 40 bis 100 Gewichtsteilen Hexamethylendiamin in einem Lösungsmittel, ausgewählt aus Wasser und Dichloräthan, bei einer Temperatur von 60 bis 70°C unter Rühren während 20 bis 60 min zur Umsetzung gebracht werden, bis die Reaktionsmischung sich zu Masse verfestigt, danach bei einer Temperatur von 60 bis 70°C, bis das Epichlorhydrin und das Hexamethylendiamin aus der Reaktionsmischung verschwunden sind, wobei das Hexamethylendiamin in Form der freien Base oder des Hydrochlorids verwendet wird; und

– das erwartete Polymerisat durch Absaugen danach Trocknen bei 50°C während 48 h gewonnen wird.

21. Verfahren nach Anspruch 20, dadurch gekennzeichnet, dass die Mengen an Polyurethan-polyol-Epichlorhydrin-Hexamethylendiamin in einem Gewichtsverhältnis von 1:3:6 bis 1:6, 6:10 stehen.

22. Polymerisat mit dreidimensionaler Struktur vom Typ vernetztes Polyurethan mit gutem Quellvermögen, dadurch gekennzeichnet, dass es gemäss dem Verfahren nach einem der Ansprüche 1 bis 21 hergestellt ist.

23. Polymerisat mit dreidimensionaler Struktur nach Anspruch 22, dadurch gekennzeichnet, dass es eine Dichte von 1,1 g/cm³ aufweist, dass es kristallin ist und bei 240°C schmilzt.

24. Polymerisat nach einem der Ansprüche 22 und 23, dadurch gekennzeichnet, dass sein Quellvermögen gegenüber Wasser von 1500 bis 4000 Gew.-% und gegenüber Plasma von 1200 bis 2500 Gew.-% ausmacht.

25. Polymerisat nach Anspruch 24, dadurch gekennzeichnet, dass es aus einem Gewichtsteil Polyurethan-Polyol (V), 4 Gewichtsteilen Epichlorhydrin und 6 Gewichtsteilen Hexamethylendiamin erhalten ist, wobei das Polyurethan-Polyol (V) durch Umsetzen eines Polyoläthers (I) mit einem Polyisocyanat (IV) in einer Weise erhalten ist, dass das numerische Verhältnis der Anzahl an freien NCO-Gruppen von (IV) zur Anzahl an freien OH-Gruppen von (I) 1:2 beträgt.

26. Insbesondere therapeutisch und kosmetisch verwendbare Zusammensetzung, dadurch gekennzeichnet, dass sie ein Polymerisat nach einem der Ansprüche 22 bis 25 als Wirkstoff enthält.

27. Zusammensetzung nach Anspruch 26, dadurch gekennzeichnet, dass das Polymerisat, das es als Wirkstoff enthält, als Quellmittel wirkt.

28. Zusammensetzung nach Anspruch 26, dadurch gekennzeichnet, dass das Polymerisat, das es als Wirkstoff enthält, mittels Wasser gequollen ist.

29. Therapeutische Zusammensetzung nach Anspruch 27, dadurch gekennzeichnet, dass sie auf oralem Weg verabreicht wird und dass das darin enthaltene Quellpolymerisat als anorexiges Mittel und Sequestriermittel von Gallensäuren wirkt.

30. Therapeutische Zusammensetzung nach Anspruch 27, dadurch gekennzeichnet, dass sie lokal verabreicht wird, insbesondere in Form eines Verbandes zur Behandlung von Wunden, und dass das darin enthaltene Quellpolymerisat vor Verabreichung in trockenem Zustand ist.

31. Kosmetische Zusammensetzung nach Anspruch 28, dadurch gekennzeichnet, dass das darin enthaltene, mittels Wasser gequollene Polymerisat als wasseranlagerndes Mittel wirkt.

32. Therapeutische Zusammensetzung nach Anspruch 28, dadurch gekennzeichnet, dass sie lokal in Form eines Verbandes zur Behandlung von Entzündungen verabreicht wird und dass das darin enthaltene gequollene Polymerisat als entzündungshemmendes Mittel wirkt.

**Patentansprüche für den Vertragsstaat: AT**

1. Verfahren zur Herstellung eines Polymerisates mit dreidimensionaler Struktur vom Typ vernetztes Polyurethan mit gutem Quellvermögen, welches Verfahren dadurch gekennzeichnet ist, dass

1) a) ein Polyoläther, ausgewählt aus der Gruppe bestehend aus den Oxyalkylenpolyolen (I) und ihren Mischungen, erhalten durch Kondensation eines zumindest zwei OH-Gruppen enthaltenden Polyols (II) mit einem Alkylenoxid (III) in einem Verhältnis von 1 zu 20 Mol von (III) pro freie OH-Gruppe von (II), mit

b) einem zumindest zwei freie NCO-Gruppen pro Molekül enthaltenden Polyisocyanat (IV) zur Umsetzung gebracht wird, welche Reaktion mit einem Überschuss an OH-Gruppen von (I) in bezug auf die Isocyanatgruppen NCO von (IV) zur Gewinnung eines Polyurethan-Polyols (V) mit freien OH-Gruppen durchgeführt wird; und

2) das so erhaltene Polyurethan-Polyol (V) mit Epichlorhydrin (VI) und einem Polyamin (VII), ausgewählt aus den Polyaminen mit zumindest zwei Aminogruppen $NH_2$ und ihren Mischungen, zur Umsetzung gebracht wird.

2. Verfahren nach Anspruch 1, dadurch gekennzeichnet, dass der Polyoläther der Stufe 1) aus einem 3 bis 6 OH-Gruppen enthaltenden Polyol (II) erhalten wird.

3. Verfahren nach Anspruch 1, dadurch gekennzeichnet, dass der Polyoläther der Stufe 1) aus einem Polyol, ausgewählt aus der Gruppe umfassend Alkylenglykole, Triole, Tetraole, Pentole, Hexole und ihre Mischungen, erhalten wird.

4. Verfahren nach Anspruch 3, dadurch gekennzeichnet, dass der Polyoläther aus einem Polyol, ausgewählt aus der Gruppe bestehend aus Sorbit, Dulcit, Pentaerythrit und Mischungen davon, erhalten wird.

5. Verfahren nach Anspruch 1, dadurch gekennzeichnet, dass das in Stufe 1) verwendete Polyisocyanat ausgewählt ist aus der Gruppe bestehend aus 2,4-Toluoldiisocyanat, 2,6-Toluoldiisocyanat, Diphenyl-methyldiisocyanat und Mischungen davon.

6. Verfahren nach Anspruch 5, dadurch gekennzeichnet, dass das Polyisocyanat ausgewählt ist aus 2,4-Toluoldiisocyanat und der gewichtsmässigen Mischung 2,4-Toluoldiisocyanat: 2,6-Toluoldiisocyanat (80:20).

7. Verfahren nach Anspruch 1, dadurch gekennzeichnet, dass die Reaktion der Stufe 1) bei einer Temperatur zwischen 20 und 150°C während zumindest 1 h unter Stickstoffatmosphäre durchgeführt wird.

8. Verfahren nach Anspruch 7, dadurch gekennzeichnet, dass die Reaktion der Stufe 1) des Polyoläthers mit Polyisocyanat bei einer Temperatur zwischen 100 und 140°C während zwei bis drei Stunden durchgeführt wird.

9. Verfahren nach einem der Ansprüche 1, 7 und 8, dadurch gekennzeichnet, dass die Reaktion der Stufe 1) mit solchen Mengen von Polyoläther (I) bzw. Polyisocyanat (IV) durchgeführt wird, dass das numerische Verhältnis der Anzahl an freien NCO-Gruppen von (IV) zur Anzahl an freien OH-Gruppen von (I) zwischen 1:3 und 3:4 beträgt.

10. Verfahren nach Anspruch 9, dadurch gekennzeichnet, dass die Reaktion der Stufe 1) mit solchen Mengen von Polyoläther (I) bzw. Polyisocyanat (IV) durchgeführt wird, dass das numerische Verhältnis der Anzahl von freien NCO-Gruppen von (IV)/Anzahl von freien OH-Gruppen von (I) zwischen 1:2 und 2:3 liegt.

11. Verfahren nach Anspruch 1, dadurch gekennzeichnet, dass nach der Stufe 1), aber vor der Stufe 2) das erhaltene Polyurethan-Polyol (V) einer Quellung mittels einer Substanz, ausgewählt aus der Gruppe Wasser, organische Lösungsmittel und Mischungen davon, unterzogen wird.

12. Verfahren nach Anspruch 11, dadurch gekennzeichnet, dass das Quellmittel Dimethylformamid ist.

13. Verfahren nach einem der Ansprüche 11 und 12, dadurch gekennzeichnet, dass die Quellung in einer Weise ausgeführt wird, dass das Polyurethan-Polyol (V) ein Volumen vom 2- bis 20fachen seines Ausgangsvolumens hat.

14. Verfahren nach Anspruch 13, dadurch gekennzeichnet, dass die Quellung in einer Weise ausgeführt wird, dass das Polyurethan-Polyol (V) ein Volumen vom 5 bis 10fachen seines Ausgangsvolumens hat.

15. Verfahren nach Anspruch 1, dadurch gekennzeichnet, dass die Reaktion der Stufe 2) (i) bei einer Temperatur zwischen 20 und 100°C während 2 min bis 2 h unter Rühren bis zum Gelieren der Reaktionsmischung und danach (ii) bei einer Temperatur von 60 bis 70°C, wobei ein Rühren nicht mehr notwendig ist, bis die Verbindungen (VI) und (VII) aus dem Reaktionsmedium verschwunden sind, durchgeführt wird.

16. Verfahren nach einem der Ansprüche 1 und 15, dadurch gekennzeichnet, dass das Polyamin der Stufe 2) insbesondere ausgewählt ist aus den aliphatischen, aromatischen und Aralkyl-Diaminen.

17. Verfahren nach Anspruch 16, dadurch gekennzeichnet, dass das Polyamin der Stufe 2) Hexamethylendiamin ist.

18. Verfahren nach Anspruch 1, dadurch gekennzeichnet, dass die Reaktion der Stufe 2) in Gegenwart eines Lösungsmittels, ausgewählt aus Wasser, Aceton, Dimethylformamid, Tetrahydrofuran, Dichloräthan, Chloroform, Tetrachlorkohlenstoff und Mischungen davon, durchgeführt wird.

19. Verfahren nach einem der Ansprüche 1 und 15 bis 18, dadurch gekennzeichnet, dass das in Stufe 2) erhaltene Produkt einem Lösungsmittelentzug unter Vakuum oder einer Absaugung unterworfen und danach während 24 bis 72 h bei 40 bis 80°C getrocknet wird.

20. Verfahren nach Anspruch 1, dadurch gekennzeichnet, dass

– ein Polyoläther (I) (der zuvor bei 120°C unter Vakuum während 3 h dehydratisiert wurde und ein Molgewicht von 100 bis 160 und einen OH-Index von 475 bis 505 aufweist) mit einem Polyisocyanat (IV), ausgewählt aus der Gruppe bestehend aus 2,4-Toluoldiisocyanat und kommerziellem Toluoldiisocyanat, enthaltend 80 Gew.-% 2,4-Isomeres und 20 Gew.-% 2,6-Isomeres, bei einer Temperatur von 100 bis 140°C während 2 bis 3 h unter Stickstoffatmosphäre in Gegenwart eines Überschusses an freien OH-Gruppen von (I) gegenüber freien NCO-Gruppen von (IV) kondensiert wird, wobei die Kondensationsreaktion derart durchgeführt wird, dass das numerische Verhältnis der Anzahl an freien NCO-Gruppen von (IV) zur Anzahl an freien OH-Gruppen von (I) zwischen 1:3 und 3:4 liegt;

– danach das so erhaltene Polyurethan-Polyol (V) mittels Dimethylformamid derart gequollen wird, dass das Polymerisat ein Volumen vom 5 bis 10fachen seines Ausgangsvolumens hat; anschliessend das Polyurethan-Polyol zerstossen wird, bei 50°C getrocknet wird und das Dimethylformamid entfernt wird;

– 1 bis 20 Gewichtsteile Polyurethan-Polyol (V) mit 30 bis 60 Gewichtsteilen Epichlorhydrin und 40 bis 100 Gewichtsteilen Hexamethylendiamin in einem Lösungsmittel, ausgewählt aus Wasser und Dichloräthan, bei einer Temperatur von 60 bis 70°C unter Rühren während 20 bis 60 min zur Umsetzung gebracht werden, bis die Reaktionsmischung sich zu Masse verfestigt, danach bei einer Temperatur von 60 bis 70°C, bis das Epichlorhydrin und das Hexamethylendiamin aus der Reaktionsmischung verschwunden sind, wobei das Hexamethylendiamin in Form der freien Base oder des Hydrochlorids verwendet wird; und

– das erwartete Polymerisat durch Absaugen danach Trocknen bei 50°C während 48 h gewonnen wird.

21. Verfahren nach Anspruch 20, dadurch gekennzeichnet, dass die Mengen an Polyurethanpolyol-Epichlorhydrin-Hexamethylendiamin in einem Gewichtsverhältnis von 1:3:6 bis 1:6, 6:10 stehen.

**Claims for the Contracting States: BE, CH, DE, FR, GB, IT, LI, LU, NL, SE**

1. Process for the preparation of a polymer of three-dimensional structure of the cross-linked polyurethane type, having a good expanding power, said process being characterized in that

1) a) a polyolether, selected from the group constituted by oxyalkylenated polyalcohol (I) and the mixtures thereof, obtained by condensation of one polyalcohol (II) containing at least two OH groups with an alkylene oxide (III) in the proportion of 1 to 20 mols of (III) per free OH group of (II), is reacted with

b) a polyisocyanate (IV) containing at least two free NCO groups per molecule, the reaction being carried out with an excess of OH groups of (I) with respect to NCO isocyanate groups (IV) to obtain a polyurethane polyalcohol (V) with free OH groups; and,

2) the polyurethane-polyalcohol thus obtained is reacted with epichlorhydrin (VI) and a polyamine (VII) selected from the polyamines having at least two NH$_2$ amino groups and the mixtures thereof.

2. Process according to claim 1, characterized in that the polyether of stage 1) is obtained from a polyalcohol II containing 3 o 6 OH groups.

3. Process according to claim 1, characterized in that the polyether of stage 1) is obtained from a polyalcohol selected from the group comprising alkyleneglycol, triols, tetraols, pentols, hexols and the mixtures thereof.

4. Process according to claim 3, characterized in that the polyether is obtained from a polyalcohol selected from the group constituted by sorbitol, dulcitol, pentaerythritol and the mixtures thereof.

5. Process according to claim 1, characterized in that the polyisocyanate used in stage 1) is selected from the group constituted by 2,4-toluenediisocyanate, 2,6-toluenediisocyanate, diphenylmethyldiisocyanate and the mixtures thereof.

6. Process according to claim 5, characterized in that the polyisocyanate is selected from 2,4-toluenediisocyanate and the weight mixture 2,4-toluenediisocyanate-2,6-toluenediisocyanate (80:20).

7. Process according to claim 1, characterized in that the reaction of stage 1) is carried out at a temperature between 20 and 150°C for at least one hour under nitrogen atmosphere.

8. Process according to claim 7, characterized in that the reaction of stage 1) of polyether with polyisocyanate is carried out at a temperature between 100 and 140°C for two to three hours.

9. Process according to any one of claims 1, 7 and 8, characterized in that the reaction of stage 1) is carried out with respective quantities of polyolether I and polyisocyanate IV such that the numerical ratio of the number of free NCO groups resulting from IV to the number of OH free groups resulting from I is between (1:3) and (3:4).

10. Process according to claim 9, characterized in that the reaction of stage 1) is carried out with respective quantities of polyether I and polyisocyanate IV such that the numerical ratio: number of free NCO groups resulting from IV to number of OH free groups resulting from 1, is between (1:2) and (2:3).

11. Process according to claim 1, characterized in that after stage 1) but before stage 2), the polyurethane-polyalcohol V thus obtained is subjected to expansion by means of a substance selected from the group constituted by water, organic solvents and the mixtures thereof.

12. Process according to claim 11, characterized in that the expanding means is dimethylformamide.

13. Process according to any one of claims 11 und 12, characterized in that expansion is carried out in such a way that polyurethane-polyalcohol V reaches between 2 and 20 times its original volume.

14. Process according to claim 13, characterized in that expansion is carried out so that polyurethane-polyalcohol V reaches between 5 and 10 times its original volume.

15. Process according to claim 1, characterized in that the reaction of stage 2) is (i) conducted at a temperature between 20 and 100°C for between 2 min and 2 hours, with stirring, until the reaction medium gels, then (ii) is continued, stirring being no longer necessary, at a temperature of 60–70°C until the compounds (VI) and (VII) have disappeared from the reaction medium.

16. Process according to any one of claims 1 and 15, characterized in that the polyamine of stage 2) is selected particularly from aliphatic, aromatic and aralkylic diamines.

17. Process according to claim 16, characterized in that the polyamine of stage 2) is hexamethylenediamine.

18. Process according to claim 1, characterized in that the polyamine of stage 2) is carried out in the presence of a solvent, selected from water, acetone, dimethylformamide, tetrahydrofurane, dichloroethane, chloroform, carbon tetrachloride and the mixtures thereof.

19. Process according to any one of claims 1, 15 and 18, characterized in that the product obtained in stage 2) is subjected to dewatering under vacuum or to suction, then dried at 40–80°C for 24 to 72 h.

20. Process according to claim 1, characterized in that:

– a polyolether I (previously dehydrated at 120°C under vacuum for 3 hours and having a molecular weight equal to 100 to 160 and an OH index of 475 to 505) is condensed with a polyisocyanate IV selected from the group constituted by 2,4-toluenediisocyanate and commercial toluenediisocyanate containing 80% by weight of 2,4 isomer and 20% by weight of 2,6 isomer, at a temperature of 100–140°C for 2–3 hours, under nitrogen atmosphere, in the presence of an excess of free OH groups obtained from I with respect to free NCO groups obtained from IV, the condensation reaction being carried out in such a way that the numerical ratio of the number of free NCO groups obtained from IV to the number of free OH groups obtained from I is comprised between (1:3) and (3:4);

– the polyurethane-polyalcohol V thus obtained is subjected to expansion by means of dimethylformamide until the polymer reaches between 5 and 10 times its original volume; the polyurethane-glycol is then ground, dried at 50°C and the dimethylformamide is eliminated;

– 1 to 20 parts by weight of polyurethane-polyalcohol V is reacted, in a solvent selected from water and dichloroethane, with 30 to 60 parts by weight of epichlorhydrin and 40 to 100 parts by weight of hexamethylenediamine, at a temperature of 60–70°C with stirring for 20 to 60 min until the reaction medium solidifies, then at a temperature of 60–70°C until the epichlorhydrin and hexamethylenediamine have disappeared from the reaction medium, hexamethylenediamine being used in the form of the free basis or hydrochlorate; and

– the expected polymer is collected by first dewatering and then drying at 50°C for 48 hours.

21. Process according to claim 20, characterized in that the quantities of polyurethanepolyalcohol-epichlorhydrin-hexamethylenediamine have a weight ratio (1:3:6) to (1:6, 6:10).

22. Polymer of threedimensional structure of the cross-linked polyurethane type, having a good swelling power, characterized in that it is prepared according to the process of any one of claims 1 to 21.

23. Polymer of threedimensional structure according to claim 22, characterized in that it has a density of 1.1 g/cm³, and in that it is crystalline and melts at 240°C.

24. Process according to any one of claims 22 and 23, characterized in that its expanding power is, towards water, from 1500 to 4000% by weight, and towards plasma from 1200 to 2500% by weight.

25. Polymer according to claim 24, characterized in that it was obtained from one part by weight of polyurethane polyalcohol V, 4 parts by weight of epichlorhydrin and 6 parts by weight of hexamethylenediamine, polyurethanepolyalcohol V being obtained by reaction of a polyether I with a polyisocyanate IV in such a way that the numerical ratio of the number of free NCO groups resulting from IV to the number of free OH groups resulting from I is (1:2).

26. Composition useful particularly in therapeutics and cosmetics, characterized in that it contains a polymer according to any one of claims 22 to 25 as active ingredient.

27. Composition according to claim 26, characterized in that the polymer contained therein as active ingredient, acts as an expanding agent.

28. Composition according to claim 26, characterized in that the polymer contained therein as active ingredient is expanded by means of water.

29. Therapeutical composition according to claim 27, characterized in that it is administered by oral route and in that the expanding polymer contained therein acts as an anorexiant and bile acids-sequestering agent.

30. Therapeutical composition according to claim 27, characterized in that it is administered by local route, particularly in the form of dressings for the treatment of wounds, and in that the expanding polymer contained therein is dry before being administered.

31. Cosmetic composition according to claim 28, characterized in that the polymer, expanded by means of water, contained therein, acts as hydrating agent.

32. Therapeutical composition according to claim 28, characterized in that it is administered by local route in the form of dressings for the treatment of inflammation, and in that the expanding polymer contained therein acts as anti-inflammatory agent.


**Claims for the Contracting State: AT**

1. Process for the preparation of a polymer of three-dimensional structure of the cross-linked polyurethane type, having a good expanding power, said process being characterized in that

1) a) a polyolether, selected from the group constituted by oxyalkylenated polyalcohol (I) and the mixtures thereof, obtained by condensation of one polyalcohol (II) containing at least two OH groups with an alkylene oxide (III) in the proportion of 1 to 20 mols of (III) per free OH group of (II), is reacted with

b) a polyisocyanate (IV) containing at least two free NCO groups per molecule, the reaction being carried out with an excess of OH groups of (I) with respect to NCO isocyanate groups (IV) to obtain a polyurethane polyalcohol (V) with free OH groups; and,

2) the polyurethane-polyalcohol thus obtained is reacted with epichlorhydrin (VI) and a polyamine (VII) selected from the polyamines having at least two NH$_2$ amino groups and the mixtures thereof.

2. Process according to claim 1, characterized in that the polyether of stage 1) is obtained from a polyalcohol II containing 3 o 6 OH groups.

3. Process according to claim 1, characterized in that the polyether of stage 1) is obtained from a polyalcohol selected from the group comprising alkyleneglycol, triols, tetraols, pentols, hexols and the mixtures thereof.

4. Process according to claim 3, characterized in that the polyether is obtained from a polyalcohol selected from the group constituted by sorbitol, dulcitol, pentaerythritol and the mixtures thereof.

5. Process according to claim 1, characterized in that the polyisocyanate used in stage 1) is selected from the group constituted by 2,4-toluenediisocyanate, 2,6-toluenediisocyanate, diphenylmethyldiisocyanate and the mixtures thereof.

6. Process according to claim 5, characterized in that the polyisocyanate is selected from 2,4-toluenediisocyanate and the weight mixture 2,4-toluenediisocyanate-2,6-toluenediisocyanate (80:20).

7. Process according to claim 1, characterized in that the reaction of stage 1) is carried out at a temperature between 20 and 150°C for at least one hour under nitrogen atmosphere.

8. Process according to claim 7, characterized in that the reaction of stage 1) of polyether with polyisocyanate is carried out at a temperature between 100 and 140°C for two to three hours.

9. Process according to any one of claims 1, 7 and 8, characterized in that the reaction of stage 1) is carried out with respective quantities of polyolether I and polyisocyanate IV such that the numerical ratio of the number of free NCO groups resulting from IV to the number of OH free groups resulting from 1 is between (1:3) and (3:4).

10. Process according to claim 9, characterized in that the reaction of stage 1) is carried out with respective quantities of polyether I and polyisocyanate IV such that the numerical ratio: number of free NCO groups resulting from IV to number of OH free groups resulting from 1, is between (1:2) and (2:3).

11. Process according to claim 1, characterized in that after stage 1) but before stage 2), the polyurethane-polyalcohol V thus obtained is subjected to expansion by means of a substance selected from the group constituted by water, organic solvents and the mixtures thereof.

12. Process according to claim 11, characterized in that the expanding means is dimethylformamide.

13. Process according to any one of claims 11 und 12, characterized in that expansion is carried out in such a way that polyurethane-polyalcohol V reaches between 2 and 20 times its original volume.

14. Process according to claim 13, characterized in that expansion is carried out so that polyurethane-polyalcohol V reaches between 5 and 10 times its original volume.

15. Process according to claim 1, characterized in that the reaction of stage 2) is (i) conducted at a temperature between 20 and 100°C for between 2 min and 2 hours, with stirring, until the reaction medium gels, then (ii) is continued, stirring being no longer necessary, at a temperature of 60–70°C until the compounds (VI) and (VII) have disappeared from the reaction medium.

16. Process according to any one of claims 1 and 15, characterized in that the polyamine of stage 2) is selected particularly from aliphatic, aromatic and aralkylic diamines.

17. Process according to claim 16, characterized in that the polyamine of stage 2) is hexamethylenediamine.

18. Process according to claim 1, characterized in that the polyamine of stage 2) is carried out in the presence of a solvent, selected from water, acetone, dimethylformamide, tetrahydrofurane, dichloroethane, chloroform, carbon tetrachloride and the mixtures thereof.

19. Process according to any one of claims 1, 15 and 18, characterized in that the product obtained in stage 2) is subjected to dewatering under vacuum or to suction, then dried at 40–80°C for 24 to 72 h.

20. Process according to claim 1, characterized in that:

– a polyolether I (previously dehydrated at 120°C under vacuum for 3 hours and having a molecular weight equal to 100 to 160 and an OH index of 475 to 505) is condensed with a polyisocyanate IV selected from the group constituted by 2,4-toluenediisocyanate and commercial toluenediisocyanate containing 80% by weight of 2,4 isomer and 20% by weight of 2,6 isomer, at a temperature of 100–140°C for 2–3 hours, under nitrogen atmosphere, in the presence of an excess of free OH groups obtained from I with respect to free NCO groups obtained from IV, the condensation reaction being carried out in such a way that the numerical ratio of the number of free NCO groups obtained from IV to the number of free OH groups obtained from I is comprised between (1:3) and (3:4);

– the polyurethane-polyalcohol V thus obtained is subjected to expansion by means of dimethylformamide until the polymer reaches between 5 and 10 times its original volume; the polyurethane-glycol is then ground, dried at 50°C and the dimethylformamide is eliminated;

– 1 to 20 parts by weight of polyurethane-polyalcohol V is reacted, in a solvent selected from water and dichloroethane, with 30 to 60 parts by weight of epichlorhydrin and 40 to 100 parts by weight of hexamethylenediamine, at a temperature of 60–70°C with stirring for 20 to 60 min until the reaction medium solidifies, then at a temperature of 60–70°C until the epichlorhydrin and hexamethylenediamine have disappeared from the reaction medium, hexamethylenediamine being used in the form of the free basis or hydrochlorate; and

– the expected polymer is collected by first dewatering and then drying at 50°C for 48 hours.

21. Process according to claim 20, characterized in that the quantities of polyurethanepolyalcohol-epichlorhydrin-hexamethylenediamine have a weight ratio (1:3:6) to (1:6, 6:10).

Fig.1

Fig. 3

Fig.2